(19) **Europäisches Patentamt / European Patent Office / Office européen des brevets**

(11) **EP 1 404 391 B2**

(12) # NEW EUROPEAN PATENT SPECIFICATION
## After opposition procedure

(45) Date of publication and mention
of the opposition decision:
**15.01.2014 Bulletin 2014/03**

(45) Mention of the grant of the patent:
**27.04.2011 Bulletin 2011/17**

(21) Application number: **02757885.5**

(22) Date of filing: **29.03.2002**

(51) Int Cl.:
*A61L 31/18* (2006.01)    *A61L 31/02* (2006.01)
*C22C 38/40* (2006.01)

(86) International application number:
**PCT/US2002/009903**

(87) International publication number:
**WO 2002/078764 (10.10.2002 Gazette 2002/41)**

(54) **PLATINUM - STAINLESS STEEL ALLOY AND RADIOPAQUE STENTS**

PLATIN - ROSTFREIE STAHLLEGIERUNG UND RÖNTGENOPAKER STENT

ALLIAGE D'ACIER INOXYDABLE ET DE PLATINE ET STENTS RADIO-OPAQUES

(84) Designated Contracting States:
**AT BE CH CY DE DK ES FI FR GB GR IE IT LI LU MC NL PT SE TR**

(30) Priority: **30.03.2001 US 823308**
**15.03.2002 US 364985 P**
**28.03.2002 US 112391**

(43) Date of publication of application:
**07.04.2004 Bulletin 2004/15**

(73) Proprietor: **Boston Scientific Limited**
**St. Michael (BB)**

(72) Inventors:
• **CRAIG, Charles, H.**
**Lakeside, CA 92040 (US)**
• **RADISCH, Herbert, R., Jr.**
**San Diego, CA 92128 (US)**
• **TROZERA, Thomas, A.**
**Del Mar, CA 92014 (US)**

• **KNAPP, David, M.**
**Saint Paul, MN 55105 (US)**
• **GIRTON, Timothy, S.**
**Edina, MN 55436 (US)**
• **STINSON, Jonathan, S.**
**Minneapolis, MN 55447 (US)**

(74) Representative: **Peterreins, Frank et al**
**Fish & Richardson P.C.**
**Highlight Business Towers**
**Mies-van-der-Rohe-Strasse 8**
**80807 München (DE)**

(56) References cited:
**EP-A- 0 604 062      EP-A2- 0 604 062**
**WO-A-99/39765      WO-A1-01/15632**
**WO-A1-01/72349      WO-A1-02/078763**
**WO-A2-95/30384      WO-A2-99/39765**
**JP-A- S55 131 157      JP-A- 2000 104 141**
**US-A- 5 609 629      US-A- 5 858 556**
**US-A- 5 858 556      US-A- 5 983 951**
**US-A- 6 027 528      US-A- 6 155 825**

**Description**

<u>Field of the Invention</u>

The present invention pertains generally to improved intravascular medical devices such as stents manufactured from a preferred alloy which is a platinum enhanced metallic alloy that is biocompatible, has good mechanical properties and is strongly radio-absorbing so that thin-walled stents of the alloy are radiopaque when implanted.

<u>Background of the Invention</u>

During invasive medical procedures, it is often necessary to accurately position an invasive medical device at a target location in the body. For this purpose, radiography is often used to periodically determine a device location in the body. To be useful, the device must be at least in part sufficiently, radiopaque. Implantation of stents in bodily lumens is typical. Others can include vena cava filters, grafts or aneurysm coils. A stent is typically delivered in an unexpanded state to a desired location in a body lumen and then expanded. The stent may be expanded via the use of a mechanical device such as a balloon, or the stent may be self-expanding.

In general, radiography relies on differences in the density of materials being imaged to provide an image contrast between materials. This is because relatively high density materials, in general, absorb greater amounts of radiation than low density materials. The relative thickness of each material normal to the path of the radiation also affects the amount of radiation absorbed. For placing stents in smaller vessel lumens, it is desirable to use a stent having a relatively thin cross section or wall thickness, which in turn makes stents of known material less radiopaque and difficult to position in a body lumen.

Mathematically, the intensity of radiation transmitted. $I_{TRANSMITTED,}$ through an object made of a particular material, is related to the intensity of the incident beam, $I_o$ by the equation:

$$I_{TRANSMITTED} = I_o \exp{-(\mu/\rho)\rho x}$$

where $\mu$ is the linear absorption coefficient of the material, $\rho$ is the density of the material, x is the thickness of the object and $\mu/\rho$ is the mass absorption coefficient. The mass absorption coefficient, $\mu/\rho$, is constant for a given material and energy of incident radiation. The mass absorption coefficient of alloys can be calculated with reasonable accuracy by the equation:

$$(\mu/\rho)_{ALLOY} = w_1(\mu/\rho)_1 + w_2(\mu/\rho)_2 + w_3(\mu/\rho)_3 \ldots$$

where $w_1$ is the weight percent of the $i^{th}$ alloying element and $(\mu/\rho)_i$ is the mass absorption coefficient for the $i^{th}$ alloying element in the pure state. Using this equation, the calculated mass absorption coefficient for 316L (an alloy which is commonly used for stents) at an incident beam energy of 100 KeV is approximately 0.392 $cm^2/gm$.

When an object in the body is successfully imaged using standard radiographic techniques, the object is said to be radiopaque. From the above discussion, it is to be appreciated that whether an object is radiopaque will depend on the thickness of the object, the material the object is made of, attenuation of radiation from surrounding materials and the energy of the radiation used to image the object. It also follows that for a given object, surrounding material and radiation energy, the material will be radiopaque at thicknesses above a certain threshold and will be non-radiopaque at thicknesses below the threshold. Importantly for the present invention, for commonly used radiation (i.e., radiation energies of about 60 - 120 KeV), 316L is only radiopaque at a stent wall thickness above approximately 0,127 mm (0.005 inches) *in vivo* Thus, stents made of 316L that have wall thicknesses thinner than approximately 0.005 inches generally cannot be successfully imaged in the body using standard radiographic techniques.

During stent placement, it is often desirable to image both the location of the medical device and the surrounding anatomy of the body. To accomplish this with high resolution, the radiation absorption of the stent relative to the surrounding tissue needs to be within a specific range. Stated another way, if the medical device is too absorbing or not absorbing enough, then an image with low resolution will result. That said, it would be desirable to have a range of materials having differing radio-absorption characteristics to allow the preparation of radiopaque stents having various sizes and thicknesses.

In addition to having the proper radio-absorption characteristics, materials that are used to manufacture stents must be biocompatible, they must be formable (i.e., have sufficient ductility and weldability to be formed into the appropriate final stent shape), and they need to provide good mechanical properties in the finished stent to hold the lumen open. Heretofore, stainless steel type 316L, which is commercially" available, has satisfied the above-described requirements, with the exception that 3.16L does not always provide the proper radio-absorption characteristics. In greater detail, 316L is readily formable, can be strengthened by work hardening, and exhibits good mechanical properties in finished stents. Furthermore, 316L is readily weldable due to it low carbon content. As for biocompatibility, 316L is corrosion resistant and has a successful history in invasive medical device applications. Thus, it would be desirable to have a range of metallic alloy compositions that retain the biocompatibility and mechanical properties of 316L, but have a range of greater radio-absorption characteristics.

EP 0 604 062 A discloses a martensitic stainless steel alloy comprised of 11.5-12.5 wt. % Cr, 9.5-10.2 wt.% Ni, 0-4.7 wt.% Mb, 0.89-5.6 wt.% of the combination of Ti and Ta, the remainder comprising Fe and trace elements, containing less than 1 wt.%.

WO 01/72349 A1 discloses a stent made of a cobalt chromium alloy containing platinum.

US 5 858 556 A discloses a stent with a multilayer structure having an outer layer and inner layer of stainless steel, and a radiopaque middle layer of tantalum, gold or platinum. Summary of the invention The invention is defined by a medical device as defined in claim 1, and by a stent as defined in claims 8, 11 and 14. Advantageous embodiments are defined in dependent claims.

The alloy is particularly useful for manufacture of intravascular medical devices. The alloy has increased radiopacity over 316L stainless steel, yet maintains physical properties such as ductibility and yield strength present in 316L stainless steel. A preferred medical device of the present invention includes a stent which is a generally tubular structure having an exterior surface defined by a plurality of interconnected struts having interstitial spaces therebetween. The generally tubular structure is expandable from a first position, wherein the stent is sized for intravascular insertion, to a second position, wherein at least a portion of the exterior surface of the stent contacts the vessel wall. The expanding of the stent is

accommodated by flexing and bending of the interconnected struts throughout the generally tubular structure.

The stent of the present invention is preferably manufactured from an alloy which has improved radiopacity relative to present utilized stainless steel alloys such as 316L alloys. The enhanced radiopacity allows production of a stent or other intravascular medical device having watt thicknesses less than about 0,127 mm (0.005 inches) while maintaining sufficient radiopacity to be radiopaque during and after placement in a body lumen. The increased radiopacity is achieved while maintaining mechanical,structural and corrosion resistance similar to alloys such as 316L. The objectives are achieved by adding platinum in preferred embodiments, to a 316L alloy by ingot or powder metallurgy, such as by vacuum induction melting, vacuum arc remelting, pressure or sintering, hot isostatic pressing, laser deposition, plasma deposition and other methods of liquid and solid phase alloying. The resulting microstucture has been found to be free from formation of harmful topologically close-packed phases by use of phase computation methodology. This was confirmed by x-ray diffraction and transmission electron microscopy.

Platinum is chosen because it is twice as dense as nickel and has an effect as an austenitizer which allows nickel content to reduced to a minimum level. It is believed this improves biocompatibility of the stent in some applications or individuals.

The stents of the present invention are preferably manufactured from an alloy of 316L with about 2 wt % to about 50 wt.% platinum. The alloy preferably includes about 11 wt.% to about 1 wt% chromium and about 5 wt.% to about 12 wt.% nickel. The alloy further includes at least about 15 wt.% iron and about 2 wt.% to about 50 wt.% platinum.

In one preferred embodiment of the present application, the alloy composition includes approximately 11.0 to 18.0 wt% chromium and approximately 8.0 to 12.0 wt.% nickel. The metallic alloy composition further includes at least approximately 35.0 wt.% iron and approximately 10 to 35 wt.% platinum. In experiments with addition of up to 30 wt.% platinum to 316L stainless steel, it has been found that radiopacity is significantly enhanced while mechanical properties are maintained. The microstucture of the alloy has been reviewed as a key in defining the material's mechanical performance and chemical stability. Matrix microstructure, grain boundary structure, second phase formation, and deformation structures were characterized as a function of the alloy additions and process conditions and correlated to the performance and stability of the resulting alloy. Optical microscopy and transmission electron microscopy were utilized to examine the effects of adding platinum on the microstructure of the commercial 316L stainless steel, and it was found that up to 30 wt.% platinum had very little effect on microstructural characteristics of the alloy, and it is believed additions up to 50% will have little effect on microstructural characteristics of the alloy, relative to 316L.

Brief Description of the Drawings

Figure 1A is a perspective view of a preferred stent of the present invention;
Figure 1B is a perspective view of an alternative stent of the present invention in a non-expanded form as mounted over a mandrel;

Figure 2 is a plan view of the stent of Figure 1B, detailing the skeletal frame structure of a preferred stent;

Figure 3 is a perspective view of the stent of Figure 1B in an expanded state with the mandrel shown to indicate expansion;

Figure 4 is a block diagram of a process used to produce a preferred alloy and foil material for use in making a preferred stent;

Figure 5 is a schematic representation of a Z-mill used in processing an alloy of the present invention;

Figure 6 depicts the microstructure of four representative alloys of the present invention;

Figure 7 depicts precipitates observed in an alloy of the present invention;

Figure 8 depicts dislocation structures from both 316L and a 12.5% platinum enhanced alloy;

Figure 9 depicts representative microstructure of alloys of the present invention after annealing;

Figure 10 depicts diffraction patterns from 316L and 30% platinum enhanced alloys;

Figure 11 graphically shows an increasing level of platinum in the austenite grains with increasing platinum content in the alloy;

Figure 12 depicts cyclic potentiodynamic polarization curves for 316L and a sample of the alloy of the present invention; and

Figure 13 graphically depicts test results for alloys of varying oxygen content.

Detailed Description of the Invention

The present invention is directed to intravascular medical devices having a platinum enhanced alloy which improves the radiopacity of an alloy in use. The alloy is particularly useful in the manufacture of implantable and/or intravascular medical devices wherein it is necessary to utilize radiography to view the device during a medical procedure or subsequent to implantation of a medical device. The alloy composition is described in detail herein along with a preferred method of manufacture. First, however, one preferred implantable medical device is described, a stent. It is, however, recognized that the present alloy could be utilized in any medical device wherein increased radiopacity is desired.

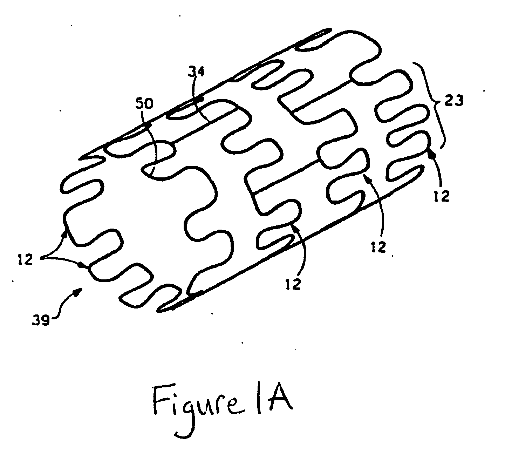

Referring now to the drawings, wherein like references refer to like elements throughout the several views, Figure 1A shows a perspective view of a stent 39 in accordance with a preferred application of the alloy of the present invention. The stent generally comprises a plurality of radially expandable cylindrical elements 12 disposed generally co-axially and interconnected by elements 34 disposed between adjacent expandable elements. The stent can be balloon expandable, self-expanding or a combination thereof. Within the cylindrical elements 12 are a series of struts or loops 50 of the stent 39. There are a series of open spaces between the struts or loops 50. This combination provides a preferred stent configuration. The cylindrical elements 12 are radially expandable due to their formation as a number of loop alterations or undulations 23 which resemble a serpentine pattern. The interconnecting elements 34 between adjacent radially expandable elements 12 are placed to achieve maximum flexibility for a stent. In the stent of Figure 1A, the stent 39 has two interconnecting elements 34 between adjacent radially expandable cylindrical elements 12 which are approximately 180 degrees apart. The next pairing of interconnecting elements 13 on one side of a cylindrical element 12 are offset by 90 degrees from the adjacent pair. This alternation of interconnecting elements results in a stent which is longitudinally flexible in essentially all directions. Other configurations for placement of interconnecting elements are possible within the scope of the present invention. However, all of the interconnecting elements of an individual stent should be secured to either the peaks or valleys of the alternating loop elements in order to prevent shortening of the stent during expansion thereof and all of the radially facing struts will have one of the specifically designed configurations.

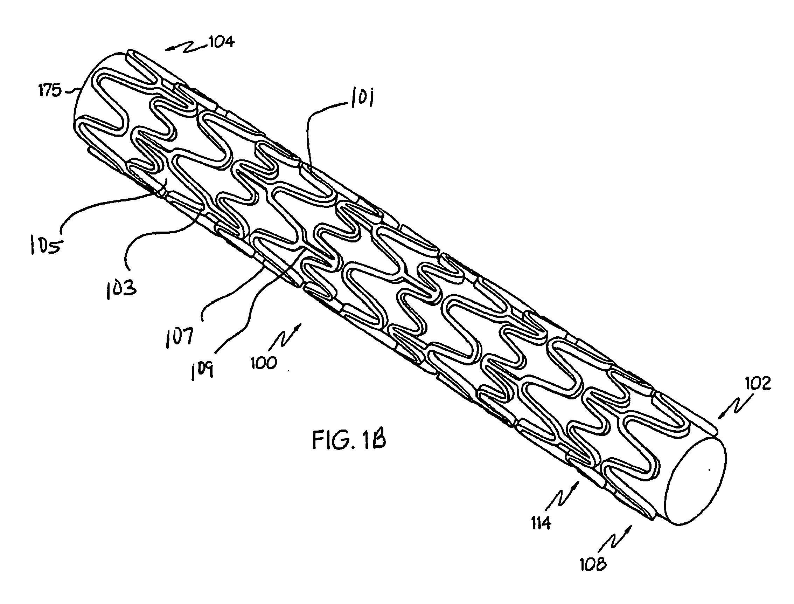

Referring now to Figure 1B, a perspective view of a stent 100, in a non-expanded form mounted on a mandrel 175, in accordance with the present invention is depicted. The stent depicted in Figure 1B is one alternative representative embodiment in which the alloy disclosed herein may be utilized. It is recognized that the alloy can be used to form any stent structure. The skeletal frame of the stent 100 preferably includes struts 101 forming a distinct, repetitive pattern. This repetitive pattern consists of multiple U-shaped curves 103. These U-shaped curves 103 form interstitial spaces 105. The U-shaped curves 103 form elements 107 which are arranged along the longitudinal axis of the stent 100 so that the U-shaped curves 103 of abutting elements 107 may be joined through interconnecting elements 109. Through the interconnecting elements 109, a continuous framework is created between multiple elements 107 forming the stent 100.

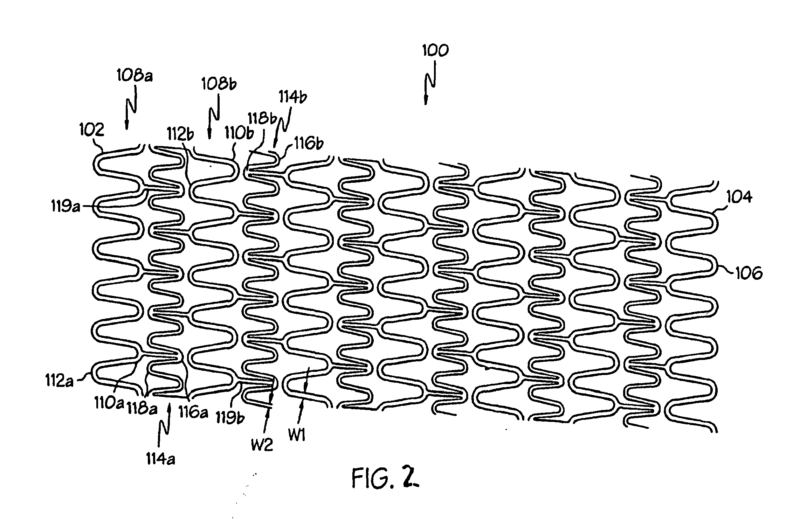

The stent of Figure 1B is depicted in planar view in Figure 2 so that the struts 101 and the framework they form can be described in more detail for preferred embodiments. Stent 100 has a proximal end 102, a distal end 104 and a flow path therethrough along a longitudinal axis 106. Stent 100 comprises a first undulating band 108a comprising a series of alternating first peaks 110a and first troughs 112a. First peaks 110a are oriented in a distal direction, and first troughs 112a are oriented in a proximal direction. First undulating band 108a is characterized by a first wavelength and a first amplitude.

Stent 100 further comprises a second undulating band 114a comprising a series of alternating second peaks 116a in a distal direction, and second troughs 118a which are oriented in a proximal direction. Second undulating band 114a is characterized by a second wavelength and a second amplitude. The second amplitude is different from the first amplitude, and the second wavelength is different from the first wavelength.

A plurality of longitudinally oriented first connectors 119a extend between first peaks 110a and second peaks 116a. Second peaks 116a, from which connectors extend, optionally have an enlarged outer radius as compared to second peaks from which no connectors extend.

Stent 100 further comprises a third undulating band 108b comprising a series of alternating third peaks 110b and third troughs 112b, and a fourth undulating band 114b comprising alternating fourth peaks 116b and fourth troughs 118b. Third peaks 110b and fourth peaks 116b are oriented in the distal direction, and third troughs 112b and fourth troughs 118b are oriented in the proximal direction. The third undulating band has a third wavelength and a third amplitude. Desirably, the third wavelength is equal to the first wavelength and the third amplitude is equal to the first amplitude. More desirably, the third band is identical in structure to the first band, as shown in Figure 2. A plurality of longitudinally oriented second connectors 126 extend between second troughs 118a and third troughs 112b. Second troughs, from which connectors extend, optionally have an enlarged outer radius relative to second troughs from which no connectors extend. The fourth undulating band has a fourth wavelength and a fourth amplitude. Desirably, the fourth wavelength is equal to the second wavelength and the fourth amplitude is equal to the second amplitude. More desirably, the fourth band is identical in structure to the second band, as shown in Figure 2. A plurality of longitudinally oriented third connectors 119b extend between third peaks 110band fourth peaks 116b. Additional undulating bands may be present in the stent. Desirably, as shown in Figure 2, the undulating bands of the stent alternate between first undulating bands of the first wavelength and first amplitude and second undulating bands of the second wavelength and second amplitude. Other arrangements of undulating bands are also within the scope of the invention. For example, one or more first undulating bands may be provided at the proximal and/or distal ends of the stent with the remaining bands being second undulating bands. Similarly, one or more second undulating bands may be provided at the proximal and/or distal ends of the stent with the remaining bands being first undulating bands.

Desirably, as shown for example in Figure 2, the first wavelength will be greater than the second wavelength. More desirably, the ratio of the first wavelength to the second wavelength in any of the embodiments disclosed herein will range from about 1.1:1 to about 5:1 and more desirably from about 1.25:1 to 2.5:1. More desirably still, the ratio will range 1.25:1 to 2:1. Another desirable ratio of wavelengths is about 1.3:1. The invention more generally contemplates any number of peaks and troughs on the first and second bands so long as the wavelengths of the two bands differ. It is also within the scope of the invention for the first wavelength to be less than the second wavelength.

Also desirably, the first amplitude is greater than the second amplitude. More desirably, the ratio of the first amplitude to the second amplitude will range from about 1.1:1 to about 4:1 and more desirably from about 1.25:1 to about 2.5:1. More desirably still, the ratio will range from about 1.25:1 to about 2:1. Even more desirably, the ratio of amplitudes of first undulating bands to second undulating bands is 1.5:1. Exemplary amplitude ratios are approximately 1.21:1, 1.29:1, 1.3:1 and 1.5:1. The invention also contemplates a stent where the first amplitude is less than the second amplitude.

As shown in Figure 2, first undulating bands 108a,b have a width $W_1$ in excess of the width $W_2$ of second undulating bands 114a,b. Desirably, the ratio of the width of the first band to the width of the second band will range from about 1:1 to about 2.5:1. Even more desirably, the ratio of the width of the first band to the width of the second band is between about 3:2 to 4:3. In another embodiment of the present invention, the first and second undulating bands may be of the same width resulting in bands of different strength. In yet another embodiment of the present invention, the second undulating bands (the smaller amplitude bands) may be wider than the first undulating bands (the larger amplitude bands). In another embodiment of the present invention, the first undulating bands may be thicker or thinner than the second undulating bands.

Desirably, as shown in Figure 2, first connectors 119 and second connectors 126 which are circumferentially adjacent, are separated by at least one second peak 116 and one second trough 118. Also desirably, first connectors 119 and second connectors 126, which are circumferentially adjacent, are separated by at least one first trough 112.

As shown in Figure 1B, the ratio of first peaks to first connectors is 2:1. The ratio of second troughs to second connectors is 3:1. Stents having other ratios of first peaks to first connectors and other ratios of second troughs to second connectors are within the scope of the invention as well. The ratio of first peaks to first connectors can equal or exceed 1:1 and more desirably equal or exceed 1.5:1, and the ratio of second troughs to second connectors will equal or exceed 1:1 and more desirably equal or exceed 3:1.

The first and second connectors are desirably straight and extend in a longitudinal direction, as shown in Figure 2. Where straight connectors are used, the desired gaps between adjacent undulating bands and the width of the bands will determine the length of the first and second connectors. Desirably, the first and second connectors will be of substantially the same length and slightly longer than the amplitude of the second undulating band. The invention also contemplates the first and second connectors being of the same length as the amplitude of the second band or substantially longer than the amplitude of the second band. The first and second connectors may also be provided in a length which differs from that of the first and second amplitudes. It is also within the scope of the invention to provide first and second connectors of different lengths from one another as shown. The first connectors may be longer than the second connectors. In another embodiment, the first connectors may be shorter than the second connectors. The stents may include additional connectors of different lengths.

The invention contemplates stents having as few as one first undulating band and one second undulating band of different wavelength and amplitude and optionally, width, connected by connectors extending from peaks on the first undulating band to peaks on the second undulating band. Desirably, however, a plurality of first undulating bands and second undulating bands alternate with one another along the length of the stent.

The rigidity of the inventive stents in the expanded state may be controlled by suitably arranging the connecting members. For example, where a stent with rigid ends and a more flexible middle portion is desired, more connecting members may be provided at the ends. Similarly, a stent with more flexible ends may be achieved by providing fewer connectors at the ends. A stent with increasing rigidity along its length may be provided by increasing the number of connectors along the length of the stent or by providing increasingly rigid undulating bands.

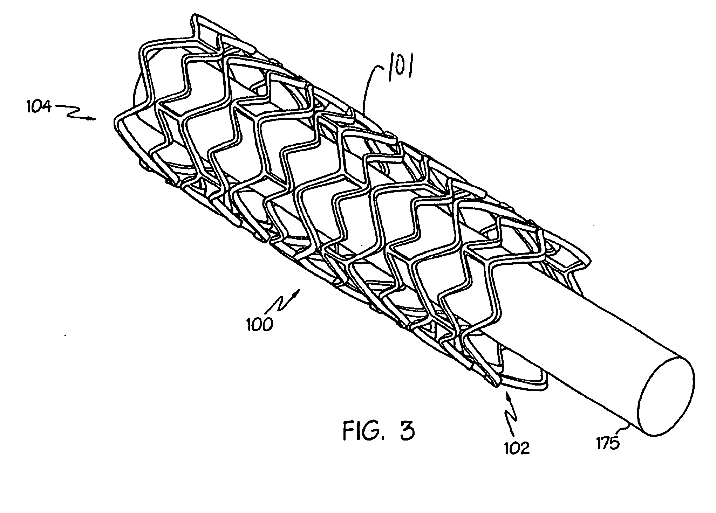

The stent of Figure 1B is shown in an expanded state in Figure 3. Bending of the struts accommodate expansion of the stent 100, with the final expanded structure resisting collapse of the lumen, when implanted, due to structural properties of the alloy of construction.

Within the range of compositions described below, the alloys used to produce the present stents are sufficiently biocompatible for use in implantable applications, have good mechanical properties and present a wide range of increased radio-absorbing properties. In greater detail, the metallic alloy composition of the present invention have slightly less chromium and nickel, by weight percent, than 316L. Further, platinum is considered to be highly biocompatible. Those skilled in the art will appreciate that because the alloys of the present invention include platinum and have levels of chromium and nickel that are below the respective levels in 316L, the alloys of the present invention are generally as biocompatible or more biocompatible as 316L. As indicated above, 316L is considered biocompatible and has a successful history of use in invasive applications.

The metallic alloy compositions of the present invention also have good mechanical properties. These mechanical properties are, in large part, due to the crystal structure of the composition. Specifically, like 316L, the platinum has face center cubic crystal structures (in its pure state). As a result, the metallic alloy compositions of the present invention have been found to have mechanical properties that are fairly similar to 316L. In particular, the metallic alloy compositions of the present invention are readily formable and can be strengthened by work hardening. In embodiments where the carbon content is controlled, the alloys of the present invention can be welded without the occurrence of grain boundary precipitates that can reduce the corrosion resistance of the alloy.

The metallic alloy compositions of the present invention also provide a wide range of increased radio-absorbing properties. Specifically, these alloys have calculated mass absorption coefficients at radiation energies of 100 KeV that are in the range of approximately 0.967 (12.5 wt%) to 1.772 (30 wt%) $cm^2/gm$, compared to the calculated mass absorption coefficient for 316L, which is only approximately 0.389 $cm^2/gm$. Because the metallic alloy compositions of the present invention strongly absorb x-ray radiation, radiopaque invasive medical devices, such as stents having thicknesses as low as 0,0381 mm (0.0015 inches), can be prepared using the compositions of the present invention.

In preferred embodiments of the present invention, the stent is manufactured from a thin-walled tube, which is then laser cut to provide the desired configuration. The tube may also be chemically etched or electrical discharge machined (EDM) to form the desired configuration. In an alternative embodiment, the stent may be made from a flat pattern which is then formed into a tubular shape by rolling the pattern so as to bring the edges together. The edges may then be joined as by welding or the like to provide a desired tubular configuration.

Metallic alloys in accordance with one embodiment of the present invention can be prepared by combining approximately 50 to approximately 95 wt.% of 316L with approximately 2 to approximately 50 wt.% of platinum. When mixed in this manner, alloys have the following range of compositions result:

Table 1

| ELEMENT | COMPOSITION WEIGHT PERCENT |
| --- | --- |
| Platinum | 2-50 |
| Carbon | 0.030 max |
| Manganese | 2.00 max |
| Phosphorous | 0.025 max |
| Sulfur | 0.010 max |

(continued)

| ELEMENT | COMPOSITION WEIGHT PERCENT |
|---|---|
| Silicon | 0.75 max |
| Chromium | 11.0 -18.0 |
| Nickel | 5.0-12.0 |
| Molybdenum | 1.4 - 2.7 |
| Nitrogen | 0.10 max |
| Copper | 0.50 max |
| Iron | Balance |

Alternatively, in accordance with the present invention, elements can be combined individually to obtain these compositions.

Example 1

Samples of the following alloys were prepared by the button melting of 316L with platinum. Only button melting, the samples were rolled into 1,527 mm (0.060-inch) thick strips and annealed, wherein alloys nos. 4,5 and 6 are covered by claim 1.

Table 2

| Alloy | Weight percent of 316L | Weight percent of platinum | Calculated mass absorption coefficient (at 100 KeV) |
|---|---|---|---|
| 1 | 90 | 10 | 0.852 cm$^2$/gm |
| 2 | 87.5 | 12.5 | 0.967 cm$^2$/gm |
| 3 | 85 | 15 | 1.082 cm$^2$/gm |
| 4 | 80 | 20 | 1.312 cm$^2$/gm |
| 5 | 75 | 25 | 1.542 cm$^2$/gm |
| 6 | 70 | 30 | 1.772 cm$^2$/gm |

Each of the alloys were analyzed using x-ray diffraction techniques, and it was determined that the primary phase (i.e., the phase of greatest weight percent) in each alloy had a face centered cubic crystal structure. Metallographic specimens were prepared and analyzed using a metallograph at 1000x for each alloy. This analysis indicated that the microstructure of each alloy consisted of equiazed and twinned austenite with no significant presence of secondary phases, intermetallics, or inclusions.

Corrosion testing was also performed on each sample including cyclic anodic polarization testing, In the forward scan, each specimen typically had an active region, passive region, and a breakdown region before scan reversal. The reverse scan always crossed the forward scan at a high potential indicating good repassivation performance of the materials. After polarization testing, the specimens were examined with a stereozoom microscopic at magnifications of 7 - 90x. The 20-30% Pt samples showed no pitting or staining. The other samples had some pitting and staining, and it is hypothesized that these were caused by voids or silicon particles that were caused during button melting.

Example 2

Tubes having 12.5 wt.% platinum (balance 316L stainless) and 30.0 wt.% platinum (balance 316L stainless) were prepared for tensile and fatigue testing. Tubes of 100 wt.% 316L stainless were prepared for comparison. To prepare the tubes, a 3-inch forged billet was machined into a hollow cylinder, and the cylinder was drawn to the final diameter of the tube. Each tube had a final outside diameter of approximately 1,778 mm (0.07 inch). After drawling, the tubes were annealed. The tubes were cut into 7-inch lengths for axial tensile testing. The average tensile test results were as follows:

Table 3

| Tubing: | 0.2% offset YS, ksi | % strain to peak load | UTS, ksi |
|---|---|---|---|
| 316L SS | 49.5 | 36.1 | 94.2 |
| 12.5% Pt | 50.0 | 40.5 | 93.2 |
| 30% Pt | 60.8 | 35.2 | 119.5 |

Axial fatigue testing was performed on the 12.5 wt.% platinum (balance 316L stainless) and the 316L stainless alloys at a maximum stress of 45 ksi. For the 12.5 wt.% platinum, fracture occurred at 575,000 cycles for one specimen, 673,000 cycles for another specimen, and the third specimen was cycled through 1,000,000 cycles without fracture. For the 316L stainless alloy, fracture occurred at 356,000 cycles for one specimen, 544,000 cycles for another specimen and the third specimen was cycled through 1,000,000 cycles without fracture.

Preferred embodiments of the present invention include expandable coronary stents made of an alloy with enhanced radiopacity to make stents more visible radiographically and more effective clinically. The enhanced radiopacity is achieved while maintaining properties similar to stainless steel used in manufacturing stents. These objectives are preferably achieved by adding a noble metal, platinum, to 316L by vacuum induction melting a commercially available alloy. Freedom of the resulting microstructure from formation of harmful topologically close packed phases was ensured by use of phase computation methodology (New PHACOMP), and confirmed by x-ray diffraction and transmission electron microscopy. Platinum was chosen since it is over twice as dense as nickel and, with approximately half its effect as an autenitizer, allows nickel content to be reduced to a minimum level.

316L alloys must meet ASTM requirements for ferrite content and inclusion content. The presence of topologically close packed phases (TCP) in such alloys is unacceptable because of their effect on alloy ductility.

New PHACOMP was utilized to determine whether TCPs would form on adding certain unspecified additional elements to a 316L matrix. At the time, the Md parameters for platinum had not been published and assumed values were utilized, based on the Md parameters available.

For Pt in a 316L base, the following average Md we calculated:

Table 4 - Md(avg) for BioDur 316L with 0 *w* to 30 *w* Pt

| BioDur 316L | 5 *w* Pt + 316L | 7.5 *w* Pt + 316L | 12.5 *w* Pt + 316L | 15 *w* Pt + 316L | 30 *w* Pt + 316L |
|---|---|---|---|---|---|
| Md(avg) = 0.913 eV | Md(avg) = 0.911 eV | Md(avg) = 0.910 eV | Md(avg) = 0.907 eV | Md(avg) = 0.906 eV | Md(avg) = 0.897 eV |

These 100 g ingots of platinum containing alloys were cast, rolled, annealed, and machined to shape. X-ray diffraction was used to determine the presence of either TCP phases or ferrite. The diffraction results showed an absence of ferrite or TCPs in the BioDur 316LS containing platinum. Radiopacity measurements showed sufficient enhancement in radiopacity of the resulting coronary stents would be provided by approximately 5.0 w Pt. Thus, it was decided to cast a 50 kg ingot in order to prepare mechanical test specimens and trial potential manufacturing processes. Later, a further series of small ingots with platinum contents up to 30 w were cast. These were then processed as before and subjected to the same analysis. No indications of TCPs were found, and radiopacity results compared well with expectations. Tubes were then manufactured from the 5 w ingot and later, from 12.5 w and 30 w ingots. These tubes were examined by both optical and transmission electron microscopy (TEM) and no indications were found of any of these alloys containing TCPs.

Processing of the alloy is controlled to alleviate concerns over dimensional control of the final thickness of the foil and over maintaining its grain size. Welded tubes made from this alloy are preferably used to fabricate stents, which are made by rolling foil into a tube, laser-welding the seam, then drawing it to the required diameter of the stent. A chemical etching process is used, which requires tubes of extremely consistent wall thickness and grain size in order to produce implant grade medical products.

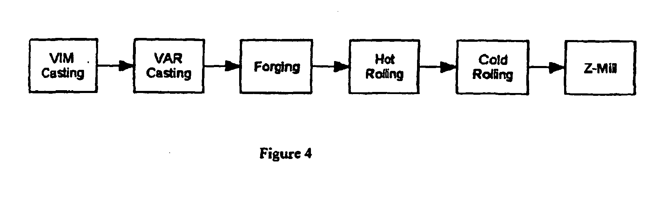

Based on constraints of thickness and grain size, a preferred process for manufacturing the foil to be used was developed. Figure 4 shows the processing steps for alloys prior to tube production and stent fabrication. The alloy is formed by Vacuum Induction Melting (VIM) a commercially available stainless steel, BioDur 316L, in rod form, along with the additional element, platinum, and any additional specified elements such as chromium and molybdenum required to maintain the alloy within the compositional specifications of F139. The alloy is refined through Vacuum Arc Remelting (VAR) and molded into an ingot. The ingot is taken through a forging process where it is formed into a billet. The billet is formed into a sheet by hot-rolling in a 2-high rolling mill and cold rolling in a 4-high rolling mill. The foil is formed by a 40% final reduction in thickness by a 20-high Sendzimir rolling mill (Z-mill).

Vacuum Induction Melting (VIM) is a metallurgical process that uses an induction furnace inside a vacuum chamber to melt and cast steel (as well as other alloys). VIM consists of heating the alloy components together in a crucible that is surrounded by a water-cooled copper coil. High frequency current passes through the coil and melts the materials within the crucible, as well as causing a powerful electromagnetic stirring action. The use of vacuum helps to minimize the amount of impurities present in the alloy by keeping oxides and other detrimental products from forming that might adversely affect its performance.

Vacuum Arc Remelting (VAR) consists of maintaining a high current DC arc between rods made from the VIM-produced alloy and a molten metal pool of the alloy that is contained in a water-cooled copper crucible. The VAR process, as with the VIM process, is kept under vacuum to maintain alloy cleanliness and eliminate impurities. The remelting process has been found to produce an ingot with good internal structure and excellent chemical homogeneity.

Forging the molded ingot into a billet is performed by compressing the ingot between two flat dies, a process also known as "upsetting". The forging process changes the microstructure of the workpiece from a cast to a wrought structure, i.e., from a chemically homogenous ingot with nonuniform grains to a wrought product with uniform grains.

Hot rolling is performed above the recrystallization temperature of the alloy. A billet from the forging process is heated and drawn through a pair of hardened steel rollers that reduces the thickness of the material over several passes to produce a plate form of the alloy. The grains initially elongate and subsequently recrystallize into smaller, more uniform grains, which provide greater strength and ductility than is provided by the metallurgical structure of the forged billet.

Cold rolling, at room temperature, is performed on the plate to reduce its thickness without allowing the grains to recrystallize. Cold rolling has the advantages of producing thin sheets with a clean surface finish, tighter dimensional tolerances, and better mechanical properties.

The final rolling of the alloy into foil requires a 40% reduction in thickness to maintain proper grain size and mechanical properties. Normal rolling mills are affected by "roll deflection", a tendency for the rolls to bend outward in response to the roll forces. This causes a crown to be formed on the rolled material in that the center is thicker than the outer edges. This effect can be countered by using a larger roll and giving it a barrel shape (camber) to offset the effects of roll deflection. Larger rolls, however, are more susceptible to roll flattening, where the rolls bulge into an oblong shape in response to the roll forces. Roll flattening can cause defects in the final material and limits the amount the material can be reduced.

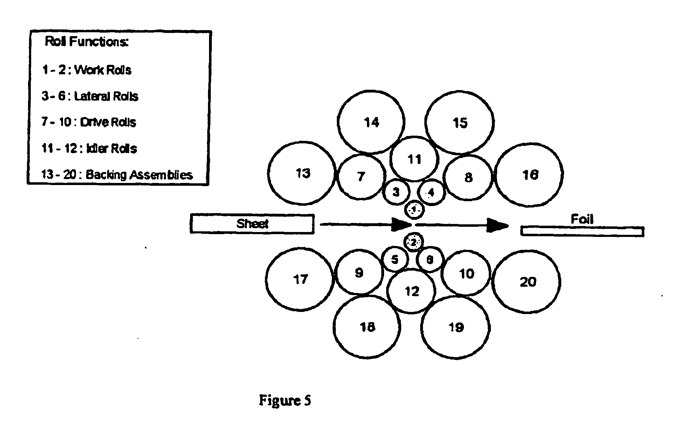

To alleviate the above cold rolling problems, it has been found useful to use a Z-mill. The Z-mill is of a class of rolling mills known as "cluster" mills (see Figure 5). Two small-diameter rolls that contact the metal are supported by a group of larger rolls. The smaller diameter rolls enable the mill to perform the 40% reduction of the material without suffering the effects of roll flattening. The smaller diameter rolls also reduce the roll force and power requirements, and help prevent horizontal spreading of the material. The larger supporting rolls prevent the working rolls from deflecting, so a consistent foil thickness can be maintained.

To test the alloy produced by the above process, BioDur 316L stainless steel rod and platinum were melted together in a VIM furnace. The ingot produced approximate dimensions of 15 cm diameter by 20 cm long. The composition of the platinum enhanced stainless steel ingot was determined and is presented in comparison to the typical composition of BioDur 316L in Table 5 below.

Table 5 - Composition of BioDur 316 L Stainless Steel and PT Enhanced Ingot

| Element | Symbol | 316 L | Pt enhanced ingot #50 |
|---|---|---|---|
| Carbon | C | 0.024 wt% | 0.023 wt% |
| Manganese | Mn | 1.80 wt% | 1.54 wt% |
| Silicon | Si | 0.44 wt% | 0.45 wt% |
| Chromium | Cr | 17.66 wt% | 18.67 wt% |
| Nickel | Ni | 14.66 wt% | 13.25 wt% |
| Molybdenum | Mo | 2.78 wt% | 2.94 wt% |
| Platinum | Pt | -- | 5.32 wt% |

To further refine the material and improve its quality, the VIM ingot was subjected to the VAR process. The ingot was secured in an evacuated chamber and allowed to act as an electrode. The amount of current passing through the material was gradually increased from 1500 A at 26 V to a maximum of 4800 A at 32 V. The ingot was then allowed to re-solidify to an approximate diameter of 15 cm and a length of approximately 20 cm.

To prepare the material for the hot-rolling process, the ingot was forged into a rectangular block (billet). The ingot was heated to 1230° C for a soak time of five hours and transferred to a forge. The material was upset through a series of compressions, reheating the material between actions of the forge to produce a billet approximately 9.5 cm x 17 cm x 22 cm.

The process of hot rolling the billet into plate form in a 2-high rolling mill took place in several stages, with a typical reduction of 10% per pass. The billet was rolled into a slab at an initial temperature of 1230° C and reheated between the subsequent passes to maintain the elevated temperature. The slab was rolled into a plate with a final thickness of 1.33 cm (0.522") and was of sufficient consistency that it was not necessary to re-flatten the material on the forge. The material was annealed at 1040° C for 14 minutes before fan-assisted cooling to room temperature.

The plate was transferred to a 4-high rolling mill and cold-rolled by an extensive series of 5% reductions with occasional fifteen-minute anneals at 1040° C. The sheet that was obtained through the first part of the cold-rolling process had a thickness of 1.63 mm (0.064"). The cold-rolled sheet was coiled and secured for a vacuum batch anneal at 950° C. The strip was cleaned and trimmed and the thickness further reduced by cold-rolling to a thickness of 0.69 mm (0.027") on the 4-high mill.

Prior to the final reduction in the Z-mill, the strip of platinum enhanced material was trimmed to a width of 15.88 cm (6.25") and strip annealed at 1065° C at approximately 2 m per minute (6 feet per minute) in a horizontal furnace. The material was then loaded onto the Z-mill and reduced to a final thickness of 0.15 mm (0.0063"). A final anneal was performed at 1050° C at approximately 1 m per minute (3 feet per minute) in the horizontal furnace.

The foil had an increased radiopacity signature compared to standard 316 L stainless steel, which makes it ideal for coronary stent applications. Further, platinum was added to 316L stainless steel without affecting material properties or biocompatibility.

Matrix microstructure, grain boundary structure, second-phase formation, and deformation structures were characterized as functions of alloy additions and process conditions, and correlated to the performance and stability of the resulting alloys. Optical microscopy and transmission electron microscopy were utilized to examine the effects of adding platinum (Pt) on the microstructure of the commercial 316L stainless steel. The results detailed below indicate that there is little change in the microstructural characteristics of 316L on additions of Pt up to 30 w.

Four materials were examined in this study: BioDur 316L stainless steel, which is commonly used in stent production, and three modified alloys containing 5 w, 12.5 w, and 30 w Pt, designated herein as 5% platinum enhanced, 12.5% platinum enhanced, and 30% platinum enhanced, respectively. Samples for analysis in the transmission electron microscope (TEM) were mechanically cut from tubes of these alloys that had been thermomechanically processed in a manner similar to that used to produce known stents. These four samples were then electropolished to electron transparency in an electrolyte consisting of 10 volume percent perchloric acid in acetic acid at 20 V and 15° C. All TEM studies were performed at an accelerating voltage of 200 kV in an FEI/Philips CM200 electron microscope equipped with a double-tilt stage for diffraction-contrast studies and with X-ray Energy Dispersive Spectroscopy (XEDS) apparatus for microchemical analysis.

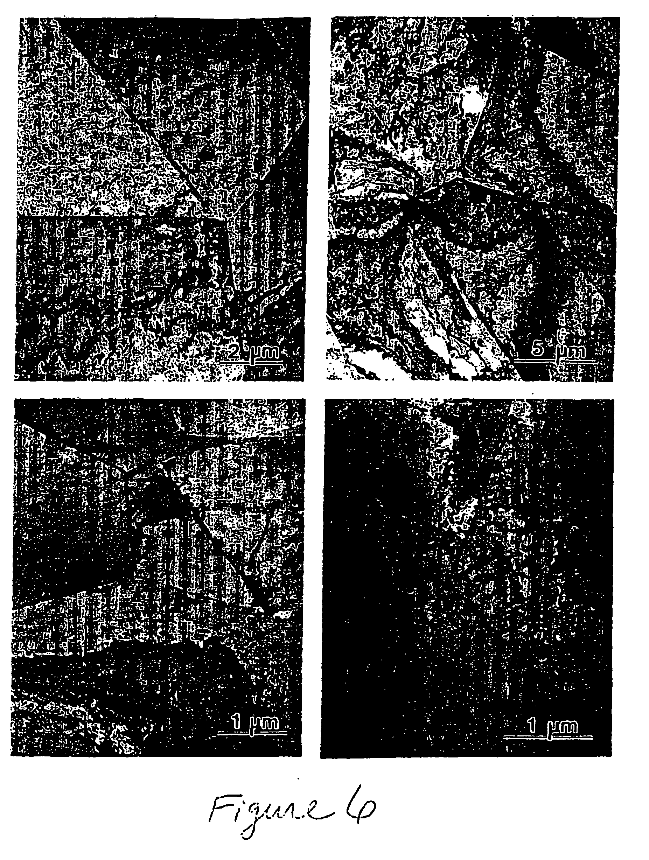

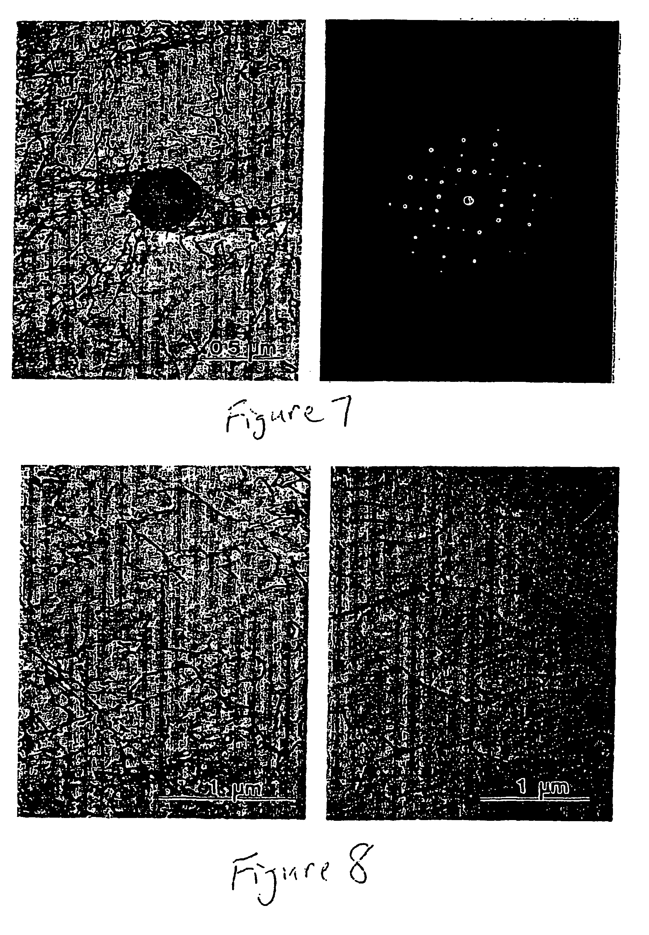

Microstructures of the four alloys examined in this study are illustrated in Figure 6. A comparison of these micrographs indicates little change in the base microstructure with Pt additions up to 30 w. In each case, the material consists of an austenitic matrix that is twinned and that contains a residual dislocation density, which matrix is dependent upon the thermomechanical treatment of the stainless steel alloy. As can be seen in these micrographs, there is no large-scale precipitation of second phases, either at the grain boundaries or within the austenite grains themselves. That is not to say, however, that there are no second phases present within these materials. Intra- and inter-granular carbide and/or oxide precipitates are occasionally observed in all the alloys examined, as illustrated for the 5% platinum enhanced alloy in Figure 7. By a combination of XEDS, chemical analysis and electron diffraction, these precipitates were identified as one of three types: $(Mo,Cr)_2C$; $(Mo,Cr)_{23}C_6$; or $(Cr,Al,Ti)_2O_3$. No Pt was detected in any of the precipitates, within the detection capabilities of the XEDS system. The number and specific type of precipitates present depend upon the impurities introduced during production and the subsequent high-temperature processing of the stent, and are common in these types of materials. But because of their low number density, their presence is not expected to significantly or adversely affect the mechanical or chemical stability of the bulk material.

The deformation mode, which is important in determining the mechanical stability and the resistance to stress corrosion cracking of the material, is principally planar in the base 316L alloy, and studies conducted suggest that it becomes increasingly more planar with Pt additions, as is illustrated by the dislocation structures from both the 316L and the 12.5% platinum enhanced alloys shown in Figure 8. Planar deformation is characterized by dislocations that are arranged in planar configurations of large groups, forming extended pile-up and multi-pole structures. Such deformation structures are common in face centered cubic (austenitic) alloys, and most likely arise in these materials from a combination of the low stacking fault energy and the short range order, or clustering, of some of the alloying elements within the austenite matrix. In these materials, type planes are the primary slip planes, and are the primary slip directions. These dislocations interact with the second phase particles within the matrix grains, but due to the low number of precipitates in the material, this interaction is not likely to influence the properties of the bulk material.

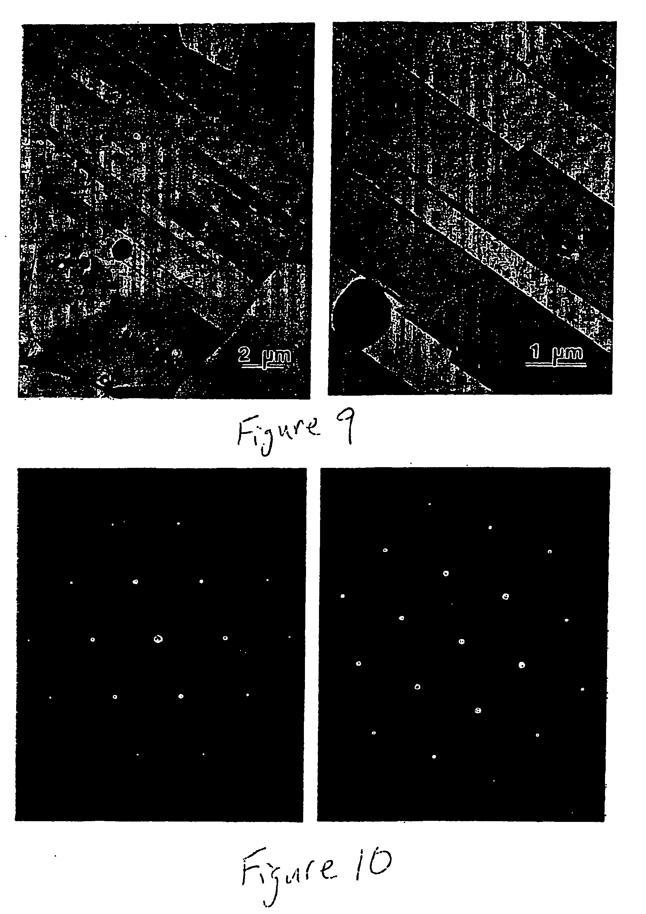

Major changes are induced in the microstructure of the 5% platinum enhanced alloy as a function of annealing temperature. For example, Figure 7 illustrates the microstructure that is typical of this alloy following heat treatment at 950° C, whereas Figure 9 show the microstructural characteristics following an anneal at 1000° C. At the higher temperature, dislocation density is significantly reduced, leaving small, clean grains, with well-defined {111}-type twins.

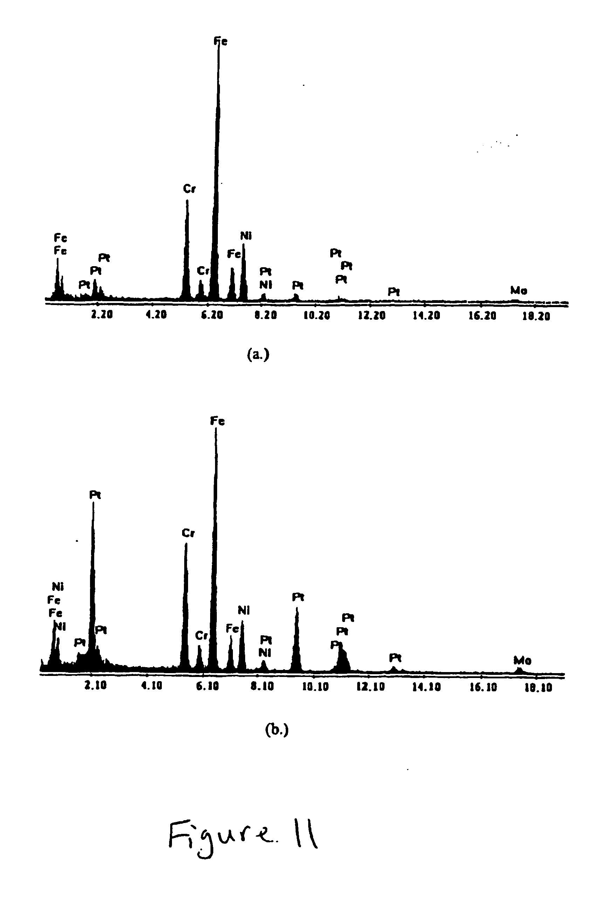

The principal effect of Pt additions on the microstructures of the platinum enhanced alloys is a slight expansion in the austenite crystal lattice as a result of the insertion of Pt atoms with a larger atomic radius than iron. Thus the lattice parameter increases from approximately 3.599 Å for the 316L alloy to approximately 3.662 Å for the 30% platinum enhanced alloy, but the platinum enhanced alloys retain their austenitic structure at room temperature. This effect is reflected in the TEM by a slight contraction in the spacing between diffraction spots in zone axis diffraction patterns of the austenite grains that contain Pt and can also be observed by a close comparison of the diffraction patterns from the 316LS alloy with the 30% platinum enhanced alloy, as shown in Figure 10. This expansion in the lattice parameter with Pt additions, combined with an absence of Pt-containing second phases found during the microchemical analyses, indicates an increasing level of Pt in the austenite grains with increasing Pt content in the alloy (Figure 11), suggesting that Pt enters into solid solution with the austenite at Pt levels of up to the limit of the samples examined, 30 w.

The results of a study on the effect of Pt additions up to 30 w on the microstructure of a commercial, austenitic stainless steel (BioDur 316L), clearly indicate Pt enters into solid solution with the alloy, causing an expansion of the face-centered cubic crystal lattice, without significantly changing the microstructural characteristics of the material.

To determine the suitability of the alloys for stent use, the effects of the addition of platinum to 316L stainless steel on the alloy's corrosion resistance in an *in vitro* synthetic solution representative of blood or blood plasma as tested. Further, tests to determine the effect of oxygen content from the melting process on the corrosion resistance of the platinum enhanced alloy were conducted.

The materials used in this study were 316 L and the same material modified by the addition of 5% platinum. Chromium and molybdenum additions were made to maintain the pitting resistance equivalent (PRE) of the alloys at PRE 26 or greater, using $PRE = [Cr] + 3.3 * [Mo]$, where [Cr] and [Mo] are the alloy chromium and molybdenum concentrations, respectively. Alloy 50 was double melted first in a vacuum and then remelted in a vacuum arc remelt (VAR) furnace. Alloy 50 was then used to make Alloy 54 and Alloy 56. Both alloys were remelted in a Hetherington (small induction) furnace under a partial pressure of argon. Alloy 54 consisted of 1 kg of Alloy 50 remelted in a new alumina ($Al_2O_3$) crucible and poured into a new conical mold. Alloy 56 consisted of 1 kg Alloy 50 plus 250 ppm aluminum plus 750 ppm calcium oxide (CaO) melted in the same crucible as Alloy 54 and poured into a conical mold. These latter alloys were designed to produce different oxygen contents.

The results of wet chemistry and inductively-coupled plasma atomic absorption spectroscopy (ICP AA) analyses of the alloys are listed in Table 6. All of the alloys had higher oxygen contents than that analyzed for 316 L.

Table 6 - Chemical Analysis of Alloys (wt%)

| Element | 316L | Alloy 37 | Alloy 38 | Alloy 50 | Alloy 54 | Alloy 56 |
|---|---|---|---|---|---|---|
| Carbon | 0.018 | NA | 0.027 | NA | NA | NA |
| Silicon | 0.45 | 0.48 | 0.47 | 0.45 | 0.45 | 0.45 |
| Manganese | 1.80 | 1.71 | 0.96 | 1.54 | 1.54 | 1.54 |
| Sulfur | 0.001 | NA | 0.0025 | NA | NA | NA |
| Phosphorus | 0.015 | NA | NA | NA | NA | NA |
| Chromium | 17.56 | 17.53 | 17.52 | 18.67 | 18.67 | 18.67 |
| Nickel | 14.79 | 13.55 | 14.2 | 13.25 | 13.25 | 13.25 |
| Molybdenum | 2.81 | 2.87 | 2.89 | 2.94 | 2.94 | 2.94 |
| Copper | 0.09 | 0.084 | 0.073 | 0.097 | 0.097 | 0.097 |
| Cobalt | 0.07 | NA | NA | NA | NA | NA |
| Aluminum | 0.009 | 0.006 | 0.009 | 0.005 | 0.005 | 0.013 |
| Nitrogen | 0.025 | NA | 0.056 | NA | NA | NA |
| Titanium | 0.002 | NA | NA | NA | NA | NA |
| Niobium | 0.013 | 0.014 | 0.015 | 0.014 | 0.014 | 0.014 |
| Vanadium | 0.07 | 0.068 | 0.058 | 0.033 | 0.033 | 0.033 |
| Platinum | NA | 4.95 | 4.78 | 5.32 | 5.32 | 5.32 |
| Oxygen | 0.0069 | NA | 0.0400 | 0.0205 | 0.0305 | 0.0100 |

NA = not applicable

The primary corrosion test procedure used to evaluate the susceptibility of all of the alloys in this study was ASTM F2129. This procedure was used to evaluate 316 L and all of the other alloys for resistance to pitting corrosion.

On the basis of the results from the ASTM F2129 procedure, additional tests were conducted on 316 L and Alloy 38 (and a similar alloy, Alloy 37). These additional test procedures included ASTM A262 - Standard Practices for Detecting Susceptibility to Intergranular Attack in Austenitic Stainless Steels - Practice E; and ASTM F746 - Standard Test Method for Pitting or Crevice Corrosion of Metallic Surgical Implant Materials.

The ASTM F2129 test method is designed to assess the corrosion susceptibility of small, metallic, implant medical devices or components using cyclic forward and reverse potentiodynamic polarization. Examples of specified devices include vascular stents. The method assesses a device in its final form and finish, as it would be implanted. The device should be tested in its entirety. While it was not the aim of this research to evaluate any finished components, this test method was still used to compare the localized corrosion performance of the alloys and 316 L. Consequently, both types of alloys were prepared in the same manner prior to testing, namely annealed with the surface ground with a 120-grit aluminum oxide abrasive. ASTM F2129 offers a selection of several simulated physiological test solutions. Ringer's solution was selected because it has the nearest composition to blood plasma. Samples of 316 L, Alloy 50, Alloy 54, and Alloy 56 were immersed in the solution after de-aerating with high purity nitrogen at 37° C. The open circuit corrosion potential ($E_{corr}$) was then measured for one hour. At the end of one hour, the cyclic potentiodynamic scan was started in the positive (noble) direction at 10 mV/min from -100 mV negative to the $E_{corr}$. The potential was reversed when the current density reached a value two decades greater than the current density at the breakdown potential ($E_b$). $E_b$ is also sometimes called the pit nucleation potential, $E_{np}$. The scan was halted when the final potential reached 100 mV negative of the $E_{corr}$ or when the current density dropped below that of the passive current density and a protection potential, $E_{prot}$, was observed.

The samples were tested in a flat cell modified to simulate the standard Avesta cell. High purity water was allowed to flow through a fiber washer at 0.6 ml/min in order to maintain a crevice-free condition. All of the tests were performed at least in duplicate.

Tests were conducted according to ASTM A262E, a procedure that is a requirement for ASTM F138 Standard Specification for Wrought 18 Chromium-14 Nickel-2.5 Molybdenum Stainless Steel Bar and Wire for Surgical Implants (316L) and ASTM F139 Standard Specification for Wrought 18 Chromium-14 Nickel-2.5 Molybdenum Stainless Steel Sheet and Strip for Surgical Implants (316 L). This practice determines the susceptibility of austenitic stainless steel to intergranular attack.

Duplicate samples of 316 L and Alloy 37 and Alloy 38 were tested in both the annealed and the sensitized heat-treated condition. The sensitized samples were heat-treated at 675° C for one hour. All of the samples were ground with 120-grit aluminum oxide abrasive. They were then embedded in copper granules and exposed for 24 hours to a boiling solution of 100g/L hydrated copper sulfate ($CuSO_4 \cdot H_2O$) and 100 ml/L of concentrated sulfuric acid ($H_2SO_4$). After exposure, the samples were bent through 180° over a mandrel with a diameter equal to the thickness of the samples. The bent samples were then examined at a 20X magnification for cracks that would be indicative of a sensitized material. No evidence of cracks were found that indicate a sensitized material.

Tests were conducted according to ASTM F746, although this procedure is not a requirement for ASTM F138 and F139. It is designed solely for determining comparative laboratory indices of performance. The results are used for ranking alloys in order of increasing resistance to pitting and crevice corrosion under the specific conditions of the test method. It should be noted that the method is intentionally designed to reach conditions that are sufficiently severe to cause breakdown of 316 L stainless steel, which is currently considered acceptable for surgical implant use, and that those alloys that suffer pitting and crevice corrosion during the more severe portion of the test do not necessarily suffer localized corrosion when placed in the human body as a surgical implant.

Three samples each of 316 L and Alloy 38 were evaluated in the annealed condition. The surface of the cylindrical sample was first ground with 120-grit aluminum oxide abrasive. It was fitted with an inert tapered collar and was immersed in a saline electrolyte, consisting of 9 g/L sodium chloride (NaCl) in distilled water, at 37° C for one hour and the corrosion potential established. Localized corrosion was then stimulated by potentiostatically polarizing the specimen to a potential of 800 mV with respect to a saturated calomel electrode (SCE). The stimulation of localized corrosion was marked by a large and generally increasing polarizing current. The potential was then decreased as rapidly as possible to a pre-selected potential either at, or more noble than, the original corrosion potential. If the alloy was susceptible to localized corrosion at the pre-selected potential, the current remained at a relatively high value and fluctuated with time. If the pit or crevice repassivated at the pre-selected potential and localized attack was halted, the current dropped to a value typical of a passive surface and decreased continuously. In the event of repassivation, the sample was repolarized and then decreased to a greater potential, and the current response observed. This was repeated until the sample did not repassivate. The critical potential for localized attack is the most noble pre-selected potential at which localized corrosion repassivated after a potential step.

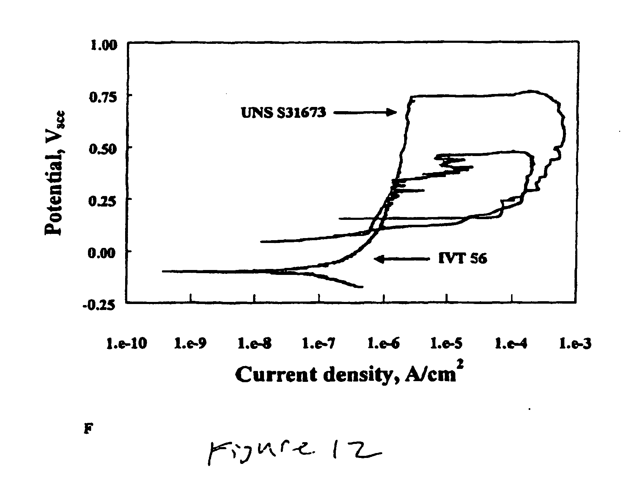

Figure 12 shows cyclic potentiodynamic polarization curves, for 316 L and Alloy 56 in de-aerated Ringer's solution, that are typical for iron-based alloys in contact with chloride solutions at moderate pH values. The curves show extended regions of passivity, a breakdown of the passive film due to the initiation and growth of pits, and a well-developed hysteresis loop. The presence of that hysteresis loop is an indication that the alloys are susceptible to localized corrosion.

The curve for Alloy 56 shown in Figure 12 is qualitatively similar to that for all of the other alloys. At the end of all experiments, pits were observed within the exposed area, and there was no indication of crevice corrosion where the samples were sealed to the test cell.

Parameters measured from the ASTM F2129 tests were $E_{corr}$, $E_b$, and $E_{prot}$. Both 316 L and the other alloys exhibited breakdown potentials more noble than their corrosion potentials, although $E_b$ for 316 L was more noble than that for the other alloys.

Table 7 summarizes the results of measured and derived values for 316 L and all of the other alloys in the ASTM F2129 tests. The data shows that the IVT alloys exhibited an $E_{corr}$ and an $E_b$ that was more active than 316 L stainless steel.

Table 7 - Results of the ASTM F2129 Tests

| Sample | $O_2$ Content Wt% | $E_{corr}$ V vs SCE | $E_b$ V vs SCE | $E_{prot}$ V vs SCE | $I_{corr}$ mA/cm$^2$ | $E_b$-$E_{corr}$ V | $E_b$-$E_{prot}$ V |
|---|---|---|---|---|---|---|---|
| 316 L | 0.007 | 0.150 | 0.742 | 0.154 | NA | 0.592 | 0.588 |
| Alloy 56 | 0.0100 | -0.098 | 0.340 | 0.103 | 0.378 | 0.438 | 0.237 |
| | | -0.079 | 0.319 | 0.100 | 0.138 | 0.398 | 0.219 |
| Alloy 50 | 0.0205 | -0.212 | 0.272 | 0.157 | 0.051 | 0.484 | 0.429 |
| | | -0.185 | 0.515 | 0.117 | NA | 0.700 | 0.632 |
| | | -0.223 | 0.204 | -0.009 | 0.192 | 0.427 | 0.213 |
| | | 0.014 | 0.452 | 0.158 | 0.022 | 0.466 | 0.610 |
| Alloy 54 | 0.0305 | -0.183 | 0.339 | 0.165 | 0.141 | 0.522 | 0.174 |
| | | 0.008 | 0.326 | 0.195 | 0.180 | 0.334 | 0.131 |

NA = not applicable

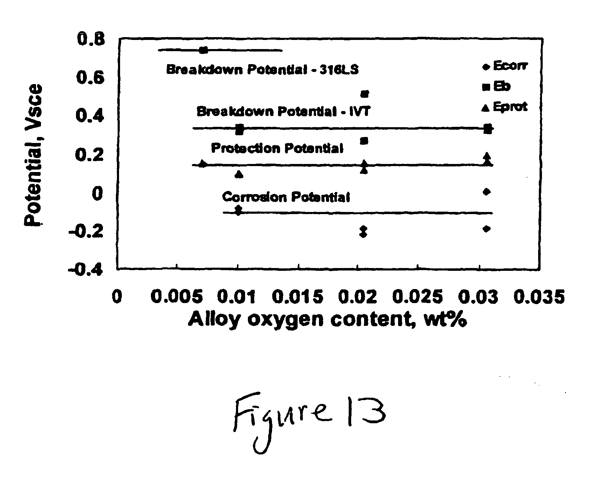

In general, local imperfections in passive films, such as caused by inclusions, increase the susceptibility of an alloy to localized corrosion. Oxygen incorporated into an alloy during the melting and fabrication process can result in the formation of oxide inclusions. Oxide inclusions appearing at the surface of a metal during corrosion tests can affect the stability of the passive film formed on stainless steels. Inclusions can become sites for preferential pit initiation and can negatively alter an alloy's resistance to pitting. It is for this reason that a series of alloys with different oxygen contents were made and tested. The results for these alloys are given in Table 7 and plotted in Figure 13. The results show that there were no observed trends in $E_{corr}$, $E_b$, or $E_{prot}$ as functions of alloy oxygen content between 0.01 and 0.0305 wt% oxygen.

The behavior of Alloy 37 and Alloy 38 was identical to that of 316L under ASTM A262E. None of the alloys exhibited any indication of sensitization. None of the samples exhibited cracks or fissures on the bend radius, which indicates that neither of the alloys was susceptible to intergranular attack.

Under ASTM F746, 316 L appeared to have better resistance to pitting and crevice attack than Alloy 38, at least as judged by the criteria of ASTM F746. That is, the critical potential for localized corrosion for 316 L, 0.200 to 0.250 $V_{SCE}$, was slightly more noble than that for Alloy 38, 0.100 to 0.150 $V_{SCE}$. The complete results are shown in Table 8.

Table 8 - results of ASTM F746 Experiments

| Sample | Exposed Area (cm$^2$) | Area Under Collar (cm$^2$) | Initial $E_{corr}$ $V_{SCE}$ | Final $E_{corr}$ $V_{CSE}$ | $E_b$ $V_{SCE}$ |
|---|---|---|---|---|---|
| 316 L | 3.62 | 0.61 | -0.177 | -0.133 | 0.200 |
| | 3.62 | 0.61 | -0.163 | -0.124 | 0.250 |
| | 3.62 | 0.61 | -0.177 | -0.117 | 0.200 |
| Alloy 38 | 3.62 | 0.61 | -0.171 | -0.093 | 0.150 |
| | 3.62 | 0.61 | -0.164 | -0.102 | 0.100 |
| | 3.62 | 0.61 | -0.221 | -0.164 | 0.150 |

Examination of the samples after testing, however, revealed that none of the samples exhibited any evidence of the localized attack, neither by crevice attack in the crevice formed by the tapered collar nor by pitting on the exposed area.

Stents of the present invention can include coatings on the alloy which incorporate therapeutic substances, alone or in a carrier which releases the therapeutic substance over time after implantation. Polymer coatings that can be utilized to deliver therapeutic substances include polycarboxylic acids; cellulosic polymers, including cellulose acetate and cellulose nitrate; gelatin; polyvinylpyrrolidone; crosslinked polyvinylpyrrolidone; polyanhydrides including maleic anhydride polymers; polyamides; polyvinyl alcohols; copolymers of vinyl monomers such as EVA; polyvinyl ethers; polyvinyl aromatics; polyethylene oxides; glycosaminoglycans; polysaccharides; polyesters including polyethylene terephthalate; polyacrylamides; polyethers; polyether sulfone; polycarbonate; polyalkylenes including polypropylene, polyethylene and high molecular weight polyethylene; halogenated polyalkylenes including polytetrafluoroethylene; polyurethanes; polyorthoesters; proteins; polypeptides; silicones; siloxane polymers; polylactic acid; polyglycolic acid; polycaprolactone; polyhydroxybutyrate valerate and blends and copolymers thereof ; coatings from polymer dispersions such as polyurethane dispersions (BAYHDROL®, etc.); fibrin; collagen and derivatives thereof; polysaccharides such as celluloses, starches, dextrans, alginates and derivatives; hyaluronic acid; and squalene emulsions.

Therapeutic substances which can be delivered from stents of the present invention include anti-thrombogenic agents such as heparin, heparin derivatives, urokinase, and PPack (dextrophenylalanine proline arginine chloromethylketone); anti-proliferative agents such as enoxaprin, angiopeptin, or monoclonal antibodies capable of blocking smooth muscle cell proliferation, hirudin, and acetylsalicylic acid; anti-inflammatory agents such as dexamethasone, prednisolone, corticosterone, budesonide, estrogen, sulfasalazine, and mesalamine; antineoplastic/ antiproliferative/anti-miotic agents such as paclitaxel, 5-fluorouracil, cisplatin, vinblastine, vincristine, epothilones, endostatin, angiostatin and thymidine kinase inhibitors; anesthetic agents such as lidocaine, bupivacaine, and ropivacaine; anticoagulants such as D-Phe-Pro-Arg chloromethyl keton, an RGD peptide-containing compound, heparin, antithrombin compounds, platelet receptor antagonists, antithrombin anticodies, anti-platelet receptor antibodies, aspirin, prostaglandin inhibitors, platelet inhibitors and tick antiplatelet peptides; vascular cell growth promotors such as growth factor inhibitors, growth factor receptor antagonists, transcriptional activators, and translational promotors; vascular cell growth inhibitors such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin; cholesterol-lowering agents; vasodilating agents; and agents which interfere with endogenous vascoactive mechanisms; anti-sense DNA and RNA; DNA coding for anti-sense RNA; tRNA or rRNA to replace defective or deficient endogenous molecules; angiogenic factors including growth factors such as acidic and basic fibroblast growth factors, vascular endothelial growth factor, epidermal growth factor, transforming growth factor $\alpha$ and $\beta$, platelet-derived endothelial growth factor, platelet-derived growth factor, tumor necrosis factor $\alpha$, hepatocyte growth factor and insulin like growth factor; cell cycle inhibitors including CD inhibitors; thymidine kinase ("TK") and other agents useful for interfering with cell proliferation; the family of bone morphogenic proteins ("BMP's"); and BMP-2, BMP-3, BMP-4, BMP-5, BMP-6 (Vgr-1), BMP-7 (OP-1), BMP-8, BMP-9, BMP-10, BMP-11, BMP-12, BMP-13, BMP-14, BMP-15, and BMP-16. Currently preferred BMP's are any of BMP-2, BMP-3, BMP-4, BMP-5, BMP-6 and BMP-7. These dimeric proteins can be provided as homodimers, heterodimers, or combinations thereof, alone or together with other molecules. Alternatively or, in addition, molecules capable of inducing an upstream or downstream effect of a BMP can be provided. Such molecules include any of the "hedgehog" proteins, or the DNA's encoding them.

## Claims

1. An intravascular medical device manufactured from a biocompatible alloy composition having a greater absorption of X-ray radiation than type 316 stainless steel, said biocompatible composition comprising:

    between 11.0 weight percent and 18.0 weight percent Chromium;
    between 5.0 weight percent and 12.0 weight percent Nickel;
    at least 15 weight percent Iron; and
    between 2.0 weight percent and 50.0 weight percent Platinum.

2. An intravascular medical device as recited in claim 1, wherein said composition further comprises Molybdenum and the weight percent of said Molybdenum is between 2.0 and 3.0.

3. An intravascular medical device as recited in claim 1, wherein said composition further comprises Carbon and said Carbon is less than 0.030 weight percent.

4. An intravascular medical device as recited in claim 1, further comprising Manganese in an amount that is greater

EP 1 404 391 B2

than zero and less than 2.0 weight percent.

5. An intravascular medical device as recited in claim 1, wherein said composition further comprises Phosphorus and said Phosphorus is less than 0.008 weight percent.

6. An intravascular medical device as recited in claim 1, wherein said composition further comprises Sulfur and said Sulfur is less than 0.004 weight percent.

7. An intravascular medical device as recited in claim 1, further comprising Silicon in an amount that is greater than zero and less than 0.75 weight percent.

8. A stent comprising:

a body portion having an exterior surface defined thereon, said body portion being expandable from a first position, wherein said body portion is sized for insertion into said lumen, to a second position, wherein at least a portion of said stent is in contact with said lumen wall, wherein the body portion is formed of a biocompatible alloy composition having a greater absorption of X-ray radiation than type 316 stainless steel, said biocompatible alloy composition including

- between 11.0 weight percent and 18.0 weight percent Chromium;
- between 5.0 weight percent and 12.0 weight percent Nickel;
- at least 15 weight percent Iron; and
- between 5.0 weight percent and 50.0 weight percent Platinum.

9. The stent as recited in claim 8, wherein the composition further comprises up to 3.0 wt.% molybdenum.

10. The stent as recited in claim 8, wherein the composition further comprises carbon in a concentration of less than 0.030 wt.%.

11. An intravascular stent adapted for treating a vessel wall comprising:

a generally tubular structure having an exterior surface defined by a plurality of interconnected struts having interstitial spaces therebetween, said generally tubular structure expandable from a first position, wherein said stent is sized for intravascular insertion, to a second position, wherein at least a portion of said stent contacts said vessel wall, said expanding of said generally tubular structure accommodated by flexing and bending of said interconnected struts, wherein the generally tubular structure is formed of a biocompatible alloy composition having a greater absorption of X-ray radiation than type 316 stainless steel, said biocompatible alloy composition including

- between 11.0 weight percent and 18.0 weight percent Chromium;
- between 5.0 weight percent and 12.0 weight percent Nickel;
- at least 15 weight percent Iron; and
- between 2.0 weight percent and 50.0 weight percent Platinum.

12. The stent as recited in claim 11, wherein the composition further comprises up to 3.0 wt.% molybdenum.

13. The stent as recited in claim 11, wherein the composition further comprises carbon in a concentration of less than 0.030 wt.%.

14. A stent having a proximal end and a distal end comprising:

a first undulating band comprising a series of alternating first peaks and first troughs, the first peaks oriented in a distal direction, the first troughs oriented in a proximal direction, the first undulating band having a first wavelength and a first amplitude;
a second undulating band comprising a series of alternating second peaks and second troughs, the second peaks oriented in a distal direction, the second troughs oriented in a proximal direction, the second undulating band having a second wavelength and a second amplitude, the second amplitude different from the first amplitude, the second wavelength different from the first wavelength;

and

at least one connector connecting first bands and second bands, wherein the stent is formed of a biocompatible alloy composition having a greater absorption of X-ray radiation than type 316 stainless steel, said biocompatible alloy composition including

- between 11.0 weight percent and 18.0 weight percent Chromium;
- between 5.0 weight percent and 12.0 weight percent Nickel;
- at least 15 weight percent Iron; and
- between 2.0 weight percent and 50.0 weight percent Platinum.

15. The stent as recited in claim 14, wherein the stent has a thickness that is less than 0,127 mm (0.005 inches).

16. The stent as recited in claim 14, wherein the composition further comprises up to 3.0 wt% molybdenum.

17. The stent as recited in claim 14, wherein the composition further comprises carbon in a concentration of less than 0.030 wt.%.

**Patentansprüche**

1. Intravaskuläre medizinische Vorrichtung hergestellt aus einer biokompatiblen Legierungszusammensetzung, die eine größere Röntgenstrahlenabsorption aufweist als Typ 316 Edelstahl, wobei die biokompatible Zusammensetzung umfasst:

zwischen 11,0 Gewichtsprozent und 18,0 Gewichtsprozent Chrom;
zwischen 5,0 Gewichtsprozent und 12,0 Gewichtsprozent Nickel;
mindestens 15 Gewichtsprozent Eisen; und
zwischen 2,0 Gewichtsprozent und 50,0 Gewichtsprozent Platin.

2. Intravaskuläre medizinische Vorrichtung gemäß Anspruch 1, wobei die Zusammensetzung weiter Molybdän umfasst und der Anteil von Molybdän zwischen 2,0 und 3,0 Gewichtsprozent liegt.

3. Intravaskuläre medizinische Vorrichtung gemäß Anspruch 1, wobei die Zusammensetzung weiter Kohlenstoff umfasst und der Kohlenstoff in weniger als 0,030 Gewichtsprozent vorliegt.

4. Intravaskuläre medizinische Vorrichtung gemäß Anspruch 1, die weiter Mangan umfasst in einer Menge, die größer als 0 ist und unter 2,0 Gewichtsprozent liegt.

5. Intravaskuläre medizinische Vorrichtung gemäß Anspruch 1, wobei die Zusammensetzung weiter Phosphor umfasst und der Phosphor in weniger als 0,008 Gewichtsprozent vorliegt.

6. Intravaskuläre medizinische Vorrichtung gemäß Anspruch 1, wobei die Zusammensetzung weiter Schwefel umfasst und der Schwefel in weniger als 0,004 Gewichtsprozent vorliegt.

7. Intravaskuläre medizinische Vorrichtung gemäß Anspruch 1, weiter umfassend Silizium in einer Menge, die größer als 0 ist und unter 0,75 Gewichtsprozent liegt.

8. Stent umfassend:

einen Körperbereich, der eine äußere Oberfläche darauf definiert hat, wobei der Körperbereich von einer ersten Position, in der der Körperbereich bemessen ist zur Einführung in das Lumen, in eine zweite Position, in der zumindest ein Teil des Stents in Kontakt mit der Lumenwand ist, aufweitbar ist, wobei der Körperbereich aus einer biokompatiblen Legierungszusammensetzung gebildet ist, die eine größere Röntgenstrahlenabsorption aufweist als Typ 316 Edelstahl, wobei die biokompatible Zusammensetzung umfasst:

- zwischen 11,0 Gewichtsprozent und 18,0 Gewichtsprozent Chrom;
- zwischen 5,0 Gewichtsprozent und 12,0 Gewichtsprozent Nickel;
- mindestens 15 Gewichtsprozent Eisen; und

- zwischen 5,0 Gewichtsprozent und 50,0 Gewichtsprozent Platin.

9. Stent gemäß Anspruch 8, wobei die Zusammensetzung außerdem bis zu 3,0 Gewichtsprozent Molybdän umfasst.

10. Stent gemäß Anspruch 8, wobei die Zusammensetzung außerdem Kohlenstoff in einer Konzentration von unter 0,030 Gewichtsprozent umfasst.

11. Intravaskulärer Stent, ausgelegt zum Behandeln einer Gefäßwand, umfassend:

eine im Wesentlichen röhrenförmige Struktur mit einer äußeren Oberfläche, die durch eine Mehrzahl von verbundenen Streben mit Zwischenräumen dazwischen definiert ist, wobei die im Wesentlichen röhrenförmige Struktur von einer ersten Position, in der der Stent bemessen ist zum intravaskularen Einführen, in eine zweite Position, in der zumindest ein Teil des Stents die Gefäßwand kontaktiert, aufweitbar ist, wobei die Aufweitung der im Wesentlichen röhrenförmigen Struktur durch Biegen und Krümmen der verbundenen Streben ausgeführt wird, wobei die im Wesentlichen röhrenförmige Struktur aus einer biokompatiblen Legierungszusammensetzung gebildet ist, die eine größere Röntgenstrahlenabsorption aufweist als Typ 316 Edelstahl, wobei die biokompatible Zusammensetzung umfasst:

- zwischen 11,0 Gewichtsprozent und 18,0 Gewichtsprozent Chrom;
- zwischen 5,0 Gewichtsprozent und 12,0 Gewichtsprozent Nickel;
- mindestens 15 Gewichtsprozent Eisen; und
- zwischen 2,0 Gewichtsprozent und 50,0 Gewichtsprozent Platin.

12. Stent gemäß Anspruch 11, wobei die Zusammensetzung außerdem bis zu 3,0 Gewichtsprozent Molybdän umfasst.

13. Stent gemäß Anspruch 11, wobei die Zusammensetzung außerdem Kohlenstoff in einer Konzentration von unter 0,030 Gewichtsprozent umfasst.

14. Stent mit einem proximalen und distalen Ende, umfassend:

ein erstes wellenförmiges Band, das eine Reihe von sich abwechselnden ersten Hochpunkten und ersten Tiefpunkten umfasst, wobei die ersten Hochpunkte in eine distale Richtung orientiert sind und die ersten Tiefpunkte in eine proximale Richtung orientiert sind, wobei das erste wellenförmige Band eine erste Wellenlänge und eine erste Amplitude aufweist;
ein zweites wellenförmiges Band, das eine Reihe von sich abwechselnden zweiten Hochpunkten und zweiten Tiefpunkten aufweist, wobei die zweiten Hochpunkte in einer distalen Richtung orientiert sind, wobei die zweiten Tiefpunkte in eine proximale Richtung orientiert sind, wobei das zweite wellenförmige Band eine zweite Wellenlänge und eine zweite Amplitude aufweist, wobei die zweite Amplitude unterschiedlich von der ersten Amplitude ist, wobei die zweite Wellenlänge unterschiedlich von der ersten Wellenlange ist; und
zumindest einen Verbinder, der die ersten und zweiten Bänder verbindet,
wobei der Stent aus einer biokompatiblen Legierungszusammensetzung gebildet ist, die eine größere Röntgenstrahlenabsorption aufweist als Typ 316 Edelstahl, wobei die biokompatible Zusammensetzung umfasst:

- zwischen 11,0 Gewichtsprozent und 18,0 Gewichtsprozent Chrom;
- zwischen 5,0 Gewichtsprozent und 12,0 Gewichtsprozent Nickel;
- mindestens 15 Gewichtsprozent Eisen; und
- zwischen 2,0 Gewichtsprozent und 50,0 Gewichtsprozent Platin.

15. Stent nach Anspruch 14, wobei der Stent eine Dicke von weniger als 0,127 mm [0,005 Inch] hat.

16. Stent nach Anspruch 14, wobei die Zusammensetzung außerdem bis zu 3,0 Gewichtsprozent Molybdän umfasst.

17. Stent nach Anspruch 14, wobei die Zusammensetzung außerdem Kohlenstoff in einer Konzentration von unter 0,030 Gewichtsprozent umfasst.

**Revendications**

1. Dispositif médical intravasculaire fabriqué à partir d'une composition en alliage biocompatible possédant une plus grande absorption aux radiations de rayons X que l'acier inoxydable de type 316, ladite composition biocompatible comportant :

   entre 11,0 pour cent en poids et 18,0 pour cent en poids de chrome ;
   entre 5,0 pour cent en poids et 12,0 pour cent en poids de nickel ;
   au moins 15 pour cent en poids de fer ; et
   entre 2,0 pour cent en poids et 50,0 pour cent en poids de platine.

2. Dispositif médical intravasculaire selon la revendication 1, dans lequel ladite composition comporte en outre du molybdène et le pourcentage en poids dudit molybdène est compris entre 2,0 et 3,0.

3. Dispositif médical intravasculaire selon la revendication 1, dans lequel ladite composition comporte en outre du carbone et ledit carbone est inférieur à 0,030 pour cent en poids.

4. Dispositif médical intravasculaire selon la revendication 1, comportant en outre du manganèse dans une quantité supérieure à zéro et inférieure à 2,0 pour cent en poids.

5. Dispositif médical intravasculaire selon la revendication 1, dans lequel ladite composition comporte en outre du phosphore et ledit phosphore est inférieur à 0,008 pour cent en poids.

6. Dispositif médical intravasculaire selon la revendication 1, dans lequel ladite composition comporte en outre du soufre et ledit soufre est inférieur à 0,004 pour cent en poids.

7. Dispositif médical intravasculaire selon la revendication 1, comportant en outre du silicone dans une quantité supérieure à zéro et inférieure à 0,75 pour cent en poids.

8. Stent comportant :

   une portion de corps ayant une surface extérieure définie sur ladite portion, ladite portion de corps étant expansible à partir d'une première position, dans laquelle ladite portion de corps est dimensionnée pour être insérée dans ledit lumen, jusqu'à une seconde position, dans laquelle au moins une portion dudit stent est en contact avec ladite paroi de lumen, dans lequel la portion de corps est formée d'une composition en alliage biocompatible possédant une plus grande absorption aux radiations de rayons X que l'acier inoxydable de type 316, ladite composition en alliage biocompatible comportant
   entre 11,0 pour cent en poids et 18,0 pour cent en poids de chrome ;
   entre 5,0 pour cent en poids et 12,0 pour cent en poids de nickel ;
   au moins 15 pour cent en poids de fer ; et
   entre 5,0 pour cent en poids et 50,0 pour cent en poids de platine.

9. Stent selon la revendication 8, dans lequel la composition comporte en outre jusqu'à 3,0 pour cent en poids de molybdène.

10. Stent selon la revendication 8, dans lequel la composition comporte en outre du carbone dans une concentration inférieure à 0,030 pourcent en poids.

11. Stent intravasculaire conçu pour traiter la paroi d'un vaisseau comportant :

   une structure généralement tubulaire ayant une surface extérieure définie par une pluralité d'entretoises interconnectées avec des espaces interstitiels entre elles, ladite structure généralement tubulaire extensible à partir d'une première position, dans laquelle ledit stent est dimensionné pour son insertion intravasculaire, jusqu'à une seconde position, dans laquelle au moins une portion dudit stent est en contact avec ladite paroi de vaisseau, ladite extension de ladite structure généralement tubulaire étant logée par flexion et courbure desdites entretoises interconnectées, dans lequel la structure généralement tubulaire est formée d'une composition en alliage biocompatible possédant une plus grande absorption aux radiations de rayons X que l'acier inoxydable de type 316, ladite composition en alliage biocompatible comportant

entre 11,0 pour cent en poids et 18,0 pour cent en poids de chrome ;
entre 5,0 pour cent en poids et 12,0 pour cent en poids de nickel ;
au moins 15 pour cent en poids de fer ; et
entre 2,0 pour cent en poids et 50,0 pour cent en poids de platine.

12. Stent selon la revendication 11, dans lequel la composition comporte en outre jusqu'à 3,0 pour cent en poids de molybdène.

13. Stent selon la revendication 11, dans lequel la composition comporte en outre du carbone dans une concentration inférieure à 0,030 pourcent en poids.

14. Stent ayant une extrémité proximale et une extrémité distale comprenant :

une première bande ondulante comportant une série de premiers sommets et premiers creux alternés, les premiers sommets étant orientés dans une direction distale, les premiers creux étant orientés dans une direction proximale, la première bande ondulante ayant une première longueur d'onde et une première amplitude ;
une seconde bande ondulante comportant une série de seconds sommets et seconds creux alternés, les seconds sommets étant orientés dans une direction distale, les seconds creux étant orientés dans une direction proximale, la seconde bande ondulante ayant une seconde longueur d'onde et une seconde amplitude, la seconde amplitude différant de la première amplitude, la seconde longueur d'onde différant de la première longueur d'onde, et ;
au moins un connecteur qui connecte les première et seconde bandes, dans lequel le stent est formé d'une composition en alliage biocompatible possédant une plus grande absorption aux radiations de rayons X que l'acier inoxydable de type 316, ladite composition en alliage biocompatible comportant
entre 11,0 pour cent en poids et 18,0 pour cent en poids de chrome ;
entre 5,0 pour cent en poids et 12,0 pour cent en poids de nickel ;
au moins 15 pour cent en poids de fer ; et
entre 2,0 pour cent en poids et 50,0 pour cent en poids de platine.

15. Stent selon la revendication 14, le stent ayant une épaisseur qui est inférieure à 0,127 mm (0,005 pouces).

16. Stent selon la revendication 14, dans lequel la composition comporte en outre jusqu'à 3,0 pour cent de molybdène.

17. Stent selon la revendication 14, dans lequel la composition comporte en outre du carbone dans une concentration inférieure à 0,030 pourcent en poids.

Figure 1A

FIG. 1B

FIG. 2

EP 1 404 391 B2

EP 1 404 391 B2

FIG. 3

23

Figure 4

Roll Functions:

1 - 2 : Work Rolls

3 - 6 : Lateral Rolls

7 - 10 : Drive Rolls

11 - 12 : Idler Rolls

13 - 20 : Backing Assemblies

Figure 5

Figure 6

Figure 7

Figure 8

Figure 9

Figure 10

(a.)

(b.)

Figure. 11

Figure 12

Figure 13

**EP 1 404 391 B2**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0604062 A
- WO 0172349 A1
- US 5858556 A